Europäisches Patentamt

European Patent Office

Office européen des brevets

(11) Veröffentlichungsnummer: **0 309 666**
**A1**

# EUROPÄISCHE PATENTANMELDUNG

(21) Anmeldenummer: **88111253.6**

(22) Anmeldetag: **13.07.88**

(51) Int. Cl.⁴: **A61B 5/08 , G01N 21/35**

(30) Priorität: **29.09.87 DE 3732835**

(43) Veröffentlichungstag der Anmeldung:
**05.04.89 Patentblatt 89/14**

(84) Benannte Vertragsstaaten:
**CH DE ES FR GB IT LI NL SE**

(71) Anmelder: **Siemens Aktiengesellschaft Berlin und München**
**Wittelsbacherplatz 2**
**D-8000 München 2(DE)**

(72) Erfinder: **Gerndt, Christian**
**Waldstrasse 14L**
**D-8192 Geretsried 1(DE)**
Erfinder: **Schneider, Hartmut, Dr.**
**Ignaz-Günther-Strasse 42**
**D-8000 München 81(DE)**

(54) Verfahren zur Überwachung des Atmens und Vorrichtung zur Durchführung des Verfahrens.

(57) Ein Verfahren zur Atem-Überwachung ermöglicht ein genaues zeitlich aufgelöstes Messen frei von elektromagnetischen Interferenzen in Patientennähe. Eine Vorrichtung zur Durchführung des Verfahrens besteht aus einem Sondenrohr (SO), welches von der ein- und ausgeatmete Atemluft (eAl, aAl) durchströmt wird und welches von einen Lichtstrahl (LSt) der Wellenlänge ($\lambda_1$) durchlaufen wird. Die mittels Messeinrichtung (ME) erfasste Intensität (I) des durch den Atemluftstrom (eAS, aAS) hindurchgegangenen Lichts ändert sich in Abhängigkeit vom $CO_2$-Gehalt der Atemluft.

FIG 1

## Verfahren zur Überwachung des Atmens und Vorrichtung zur Durchführung des Verfahrens

Die Erfindung betrifft ein Verfahren zur Überwachung des Atmens, insbesondere für die intensive Überwachung eines Patienten, nach dem Oberbegriff des Patentanspruchs 1 sowie eine Vorrichtung zur Durchführung des Verfahrens.

Verfahren der genannten Art können in der Medizin, insbesondere in der Humanmedizin, wertvolle Hilfe bei der Patientenüberwachung, beispielsweise bei operativen Eingriffen und auf Intensivstationen, leisten. Anhand von Atemfrequenz, -volumen und $CO_2$-Konzentration kann eine Vitalitätsdiagnose erstellt werden.

Bei einem bisherigen Verfahren erfolgt die Messung der Gaskonzentrationen auf elektrochemischem Wege. Dabei kommt es auf die Registrierung kleiner Potentialdifferenzen an, was unter Umständen durch elektromagnetische Störfelder erheblich erschwert wird. Zudem erfolgt die Einstellung elektrochemischer Potentiale in der Regel zeitlich verzögert.

Die $CO_2$-Konzentration und ihre zeitliche Änderung kann auch durch Messen der Infrarot-Absorption im Wellenlängenbereich einer $CO_2$-Bande erfolgen, beispielsweise in der sehr intensiven Grundschwingungsbande bei 4,3 µm Wellenlänge und in den mittelstarken Kombinationsschwingungsbanden um 2,77 und 2,69 µm (siehe dazu Molecular Spectra and Molecular Structure, Vol. II, Van Nostrand, New York, 1945, S. 272 ff). In diesem Fall muß ein Teil der Atemluft über eine Schlauchleitung in die Gasküvette eines Spektrometers gepumpt werden, was mit einem Verlust an zeitlicher Auflösung erkauft werden muß. Dieses Verfahren erfordert zudem einen erheblichen apparativen Aufwand in Patientennähe.

Aufgabe der Erfindung ist es, ein einfaches Verfahren der eingangs genannten Art anzugeben, das ein genaues zeitlich aufgelöstes Messen frei von elektromagnetischen Interferenzen mit minimalen Aufwand in Patientennähe ermöglicht.

Diese Aufgabe wird durch die im kennzeichnenden Teil des Patentanspruchs 1 angegebenen Merkmale gelöst.

Vorzugsweise wird die Intensität des durch den Atemluftstrom hindurchgegangenen Lichts gemessen (Anspruch 2), wobei es zweckmäßig ist, diese Intensität zumindest bei einer Wellenlänge zu messen, bei welcher das Gas das Licht in Abhängigkeit von seiner Konzentration in der Atemluft mehr oder weniger absorbiert (Anspruch 3).

Vorteilhaft ist es dabei, die Intensität des durch den Atemluftstrom hindurchgegangenen Lichts bei einer Wellenlänge zu messen, bei der das Gas das Licht absolut oder wenigstens relativ maximal absorbiert. Bei einer bevorzugten Ausführungsform dieses Verfahrens wird die Intensität des durch den Atemluftstrom hindurchgegangenen Lichts bei einer Wellenlänge im Infrarotbereich gemessen, bei der $CO_2$ das Licht absolut oder zumindest relativ maximal absorbiert (Anspruch 5).

Im Hinblick auf eine später beschriebene Vorrichtung zur Durchführung des erfindungsgemäßen Verfahrens, bei welcher Glasfaser-Lichtwellenleiter verwendet werden, ist es besonders vorteilhaft, die Intensität bei der Wellenlänge 2,69 µm oder 2,70 µm zu messen (Anspruch 6). Zwar könnte auch im Grundschwingungsbereich gemessen werden, der bei einer Wellenlänge von mehr als 4 µm liegt, jedoch sind niedrigdämpfende Fasern, die bei einer Länge von mehr als 1 m einsetzbar sind, heute noch nicht verfügbar. Schwermetallfluorid-Glasfasern (siehe dazu J. Lightwave Techn., LT-2 (1984) S. 566-586) haben jedoch in der Nähe der Kombinationsschwingungsbanden bei 2,77 und 2,69 µm bei einer Länge von mehr als 1 m vorteilhafterweise eine ausreichend niedrige Dämpfung.

In der Atemluft sind mindestens zwei Gase enthalten, die im Infrarotbereich absorbieren. Es sind dies $CO_2$ und $H_2O$, eventuell auch weitere, wie $N_2O$ oder $C_2H_2OH$, deren Spektren sich im Oberschwingungsbereich teilweise überlappen. In einem derartigen Fall ist es zweckmäßig, das erfindungsgemäße Verfahren gemäß Anspruch 7 durchzuführen, wobei es im Fall des Anspruchs 6 zweckmäßig ist, die Intensität etwa bei der weiteren Wellenlänge 2,60 µm zu messen (Anspruch 8).

Eine vorteilhafte Vorrichtung zur Durchführung eines erfindungsgemäßen Verfahrens, bei der eine optische Faser verwendet wird, ist im Anspruch 9 angegeben. Bei dieser erfindungsgemäßen Vorrichtung sind Meßstelle und Nachweisgerät voneinander getrennt. Es sei in diesem Zusammenhang darauf hingewiesen, daß aus Electron. Lett. 15 (1979) S. 749-751 unter der Bezeichnung "Remote Infrared Spectroscopy" bereits eine Vorrichtung bekannt ist, die zur Registrierung von Kohlenwasserstoffen und $NO_x$-Gasen dient und im Wellenlängenbereich um 1,6 µm mit Quarzglasfasern arbeitet.

Bevorzugte und vorteilhafte Ausgestaltungen der erfindungsgemäßen Vorrichtung gehen aus den Ansprüchen 10 bis 13 hervor.

Wie bei der aus Electron. Lett. 15 (1979) S. 749-751 bekannten Vorrichtung ist es auch bei der erfindungsgemäßen Vorrichtung vorteilhaft, wenn gemäß Anspruch 14 die Entfernung des Meßgeräts und der Lichtquelle vom Atemluftstrom größer als 1 m ist.

Damit dies im Fall der Messung der Konzentra-

tion von $CO_2$ bei der Wellenlänge um 2,69 μm herum möglich ist, besteht bei der erfindungsgemäßen Vorrichtung die optische Faser gemäß Anspruch 15 aus einer Schwermetallfluorid-Glasfaser, insbesondere gemäß Anspruch 16 aus einer teflonummantelten Bariumfluorozirkonat-Glasfaser, deren Dämpfungsspektrum um 2,5 μm in der Nähe des $CO_2$-Absorptionsmaximums bei 2,69 μm liegt. Damit lassen sich problemlos Abstände von 50 m zwischen der Lichtquelle bzw. dem Meßgerät und dem Atemluftstrom überbrücken.

Eine weitere Ausgestaltung der erfindungsgemäßen Vorrichtung, bei der das Atmen mehrerer Patienten problemlos überwacht werden kann, ist im Anspruch 17 angegeben. Bei dieser Ausgestaltung besteht die Möglichkeit, mehrere Sonden im Multiplex-Betrieb auszulesen, wodurch eine wesentlich verbesserte Ausnutzung des Meßgeräts, beispielsweise eines Spektrometers, ermöglicht ist.

Durch die Erfindung ist auch generell ein neuartiges Verfahren zur Messung der Konzentration eines in Atemluft enthaltenen Gases gegeben, das im Anspruch 18 angegeben ist.

Die Erfindung wird anhand der Figuren in der nachfolgenden Beschreibung näher erläutert. Von den Figuren zeigen:

Figur 1 in schematischer Darstellung eine Ausführungsform einer Vorrichtung zur Durchführung des Verfahrens,

Figur 2 das Infrarot-Spektrum von $CO_2$ und $H_2O$ im Oberschwingungsbereich, wobei auf der Abszisse die Wellenlänge in nm und auf der Ordinate die Transmission in % angegeben ist,

Figur 3 das Dämpfungsspektrum einer Fluoridglasfaser mit Teflonmantel eine mit der Vorrichtung nach Figur 1 gemessene Atemfrequenzkurve über der Zeit, wobei auf der Ordinate die relative Transmission des den Atemluftstrom durchstrahlenden Lichts angegeben ist,

Figur 4 eine mit der Ausführungsform nach Figur 1 aufgenommene Atemfrequenzkurve, und

Figur 5 in schematischer Darstellung eine modifizierte Ausführungsform nach Figur 1.

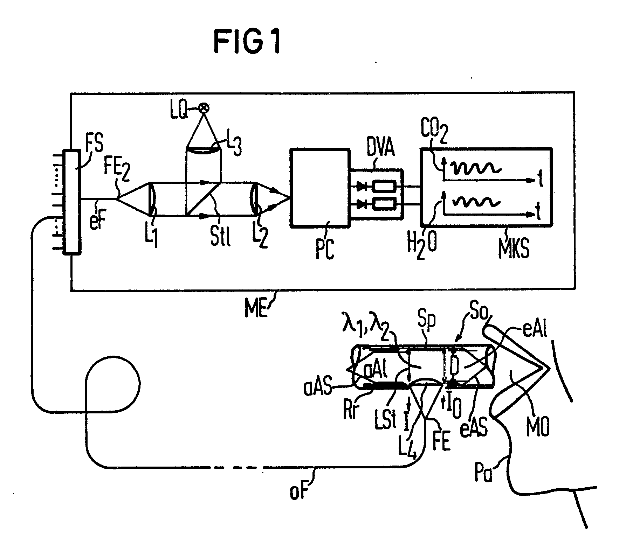

Die Vorrichtung nach Figur 1 dient beispielsweise zur Messung der Konzentration von $CO_2$ in der Atemluft und besteht im wesentlichen aus der vor der Mundöffnung MO eines Patienten Pa angeordneten Sonde So und der Meßeinrichtung ME, die durch eine Glasfaser oF aus teflonbeschichtetem Bariumfluorozirkonat mit der Sonde So verbunden ist.

Die Sonde So besteht aus einem Glasröhrchen Rr mit einem Innendurchmesser D, durch das die ein- und ausgeatmete Atemluft eAl bzw. aAl teilweise strömt, und das einen ein- und ausgeatmeten Atemluftstrom eAS, aAS des Druchmessers D definiert.

Dieser Atemluftstrom eAS bzw. aAS wird von einem Lichtstrahl LSt senkrecht zum Atemluftstrom eAS, aAS durchstrahlt, der die Wellenlänge $\lambda_1$ = 2,70 μm und die weitere Wellenlnge $_2$ = 2,60 μm aufweist.

Der Lichtstrahl LSt wird einem nahe bei dem Atemluftstrom eAS bzw. aAS befindlichen Ende FE der von einer Lichtquelle LQ in der Meßeinrichtung ME zur Sonde So führenden Faser oF ausgekoppelt und von einer optischen Linse $L_4$ kollimiert. Der kollimierte Strahl LSt durchstrahlt den Atemluftstrom eAS bzw. aAS in Richtung eines auf der anderne Seite dieses Stroms eAS bzw. aAS angeordneten Spiegels Sp. Der am Spiegel Sp reflektierte kollimierte Strahl LSt durchstrahlt den Atemlufsttrom eAS bzw. aAS ein zweites Mal senkrecht und wird von der optischen Linse $L_4$ auf das eine Ende FE der Faser oF fokussiert und dort in diese Faser oF eingekoppelt, von der er zur Meßeinrichtung ME geleitet wird.

Das in den Atemluftsrom eAS bzw. aAS eingestrahlte Licht der Wellenlänge 2,70 μm wird in Abhängigkeit von der Konzentration des $CO_2$ in der Atemluft absorbiert, so daß die Intensitt $I_0$ dieses eingestrahlten Lichts mehr oder weniger geschwächt wird und dieses Licht nach Durchgang durch den Atemluftstrom eAS bzw. aAS mit der kleineren Intenstiät I aus diesem Strom austritt.

In der Meßeinrichtung ME ist neben der Lichtquelle LQ eine Einrichtung PC zur wellenlängenselektiven Registrierung, beispielsweise ein Polychromator, angeordnet.

Da in der Atemluft mindestens zwei im Infrarotbereich absorbierende Gase $CO_2$ und $H_2O$ und eventuell auch weitere solche Gase wie $N_2O$ oder auch $C_2H_5OH$ vorhanden sind, deren Spektren sich im Oberschwingungsbereich teilweise überlappen, ist die Registrierung bei mindestens zwei Wellenlängen erforderlich.

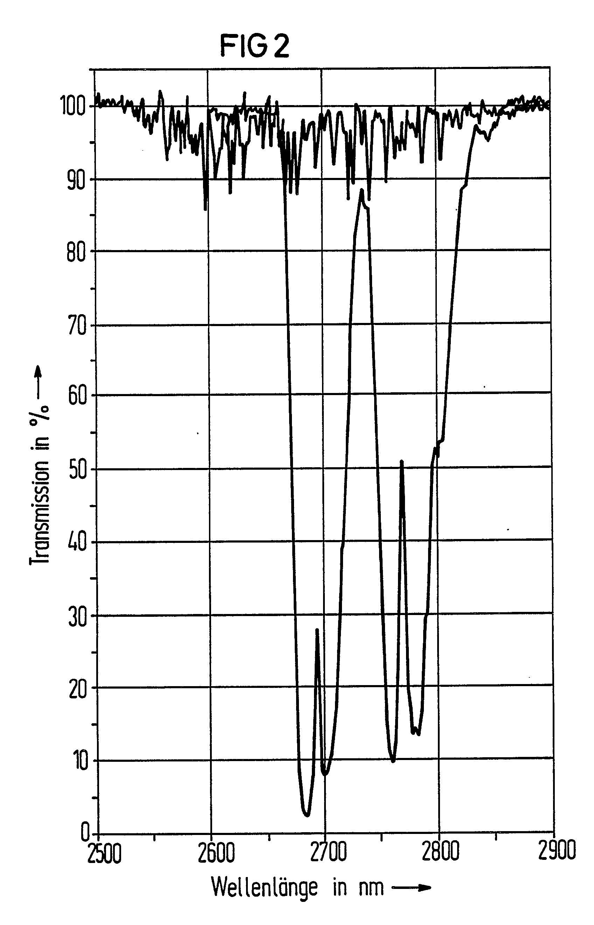

Die Figur 2 zeigt beispielsweise ein Infrarotspektrum von $CO_2$ und $H_2O$ in diesem auf der Abszisse aufgetragenen Oberschwingungsbereich zwischen der Wellenlänge 2500 nm und 2900 nm, wobei auf der Ordinate dieser Figur die Transmission aufgetragen ist.

Die Extinktion $E_1$ der Atemluft bei $\lambda_1$ = 2,70 μm mit typisch 4 % Kohlendioxid und 3 % Wasserdampf wird danach zu etwa gleichen Teilen $CO_2$ und $H_2O$ hervorgerufen. Daher wird gleichzeitig die Extinktion $E_2$ der Atemluft bei einer weiteren Wellenlänge $\lambda_2$ verfolgt, bei der $CO_2$ schwächer oder nicht absorbiert, beispielsweise bei der Wellenlänge $\lambda_2$ = 2,60 μm. Den $CO_2$-Partialdruck $p_1$ und $H_2O$-Partialdruck $p_2$ erhält man nach

$$E_1 = [\alpha_1(\lambda_1) \cdot p_2 + \alpha_2(\lambda_1) \cdot p_2] \cdot d$$
$$e_2 = [\alpha_1(\lambda_2) \cdot p_1 + \alpha_2(\lambda_2) \cdot p_2] \cdot d$$
mit

$\alpha_1(2,70 \, \mu m) = 0,1087 \; atm^{-1}cm^{-1}$

$\alpha_2(2,70 \, \mu m) = 0,06 \; atm^{-1}cm^{-1}$

$\alpha_1(2,60 \, \mu m) = 0$

$\alpha_2(2,60 \, \mu m) = 0,06 \; atm^{-1}cm^{-1}$

damit die Registrierung bei den mindestens zwei Wellenlängen $\lambda_1$ und $\lambda_2$ erfolgen kann, ist auf der Ausgangsseite der Einrichtung PC zur wellenlängenselektiven Registrierung ein Detektor/Verstärker-Array angeordnet, von dem aus eine Mehrkanal-x/t-Schreiber MKS betrieben wird, der die $CO_2$-Konzentration und die $H_2O$-Konzentration in der Atemluft über der Zeit t aufzeichnet.

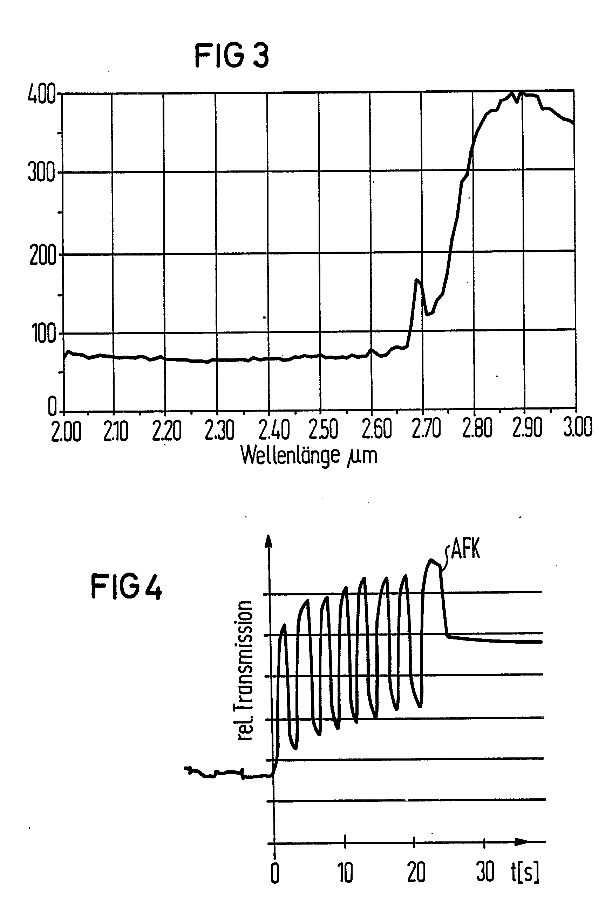

Wesentlich ist, daß die Extinktions- bzw. Absorptionsmessung mit Hilfe von für Infrarotlicht transparenten Glasfasern zur Trennung von Meßstelle und Nachweisgerät erfolgt. Das Dämpfungsspektrum der vorzugsweise verwendeten, teflonummantelten Bariumfluorozirkonat-Glasfaser zeigt die Figur 3, bei der auf der Abszisse die Wellenlänge $\lambda$ und auf der Ordinate die Dämpfung aufgetragen ist. Nach diesem Spektrum liegt das Dämpfungsminimum dieser Faser um 2,5 $\mu m$ herum und damit in der Nähe des Absorptionsmaximums von $CO_2$ bei 2,69 $\mu m$ (siehe Figur 2).

Die Figur 4 zeigt die bei 2,70 $\mu m$ gemessene Atemfrequenzkurve eines Probanden, wie sie ähnlich beispielsweise von dem x/t-Schreiber MKS aufgezeichnet wird. Die in Figur 4 dargestellte Atemfrequenzkurve AFK ist der Summe aus der $CO_2$- und $H_2O$-Extinktionsänderung proportional. Erwartungsgemäß sinkt die auf der Ordinate der Figur 4 aufgetragene relative Transmission in der Ausatmungsphase und erreicht ihr Minimum gegen Ende dieser Phase. Ferner ist zu erkennen, daß der Proband nicht ganz gleichmäßig mit einer Frequenz von etwa 17 $min^{-1}$ geatmet hat.

Bei der Ausführungsform nach Figur 1 dient dieselbe Faser oF zur Hin- und Rückleitung des von der Lichtquelle LQ ausgesandten Lichts. Im Meßgerät ME ist dazu ein Strahlteiler Stl im Strahlengang des von der Lichtquelle LQ ausgesandten und von einer Linse $L_3$ kollimierten Lichts angeordnet, der einen Teil dieses Lichts durchläßt und einen anderen Teil in Richtung einer Linse $L_1$ umlenkt, die diesen anderen Teil auf ein Ende $FE_2$ einer Ein-/Ausgangsfaser eF eines Faserumschalters FS fokussiert, in die dieser andere Teil eingekoppelt wird. An den Faserumschalter FS ist andererseits die Faser oF angeschlossen, die diesen anderen Teil des Lichts dem Atemluftstrom eAS bzw. aAS zuleitet. Das durch den Atemluftstrom eAS bzw. aAS hindurchgegangene Licht wird durch die Faser oF dem Faserumschalter FS zugeleitet und aus dem Ende $FE_2$ von dessen Ein-/Ausgangsfaser eF ausgekoppelt. Das ausgekoppelte Licht wird durch die Linse $L_1$ kollimiert und trifft auf den Strahlteiler Stl, der einen Teil dieses Lichts durchläßt, der von einer Linse $L_2$ auf die Einrichtung PC fokussiert wird.

Durch die Verwendung des Faserumschalters FS besteht die Möglichkeit, mehrere Sonden So im Multiplexbetrieb auszulesen, wodurch eine wesentlich verbesserte Ausnutzung der Meßeinrichtung ME ermöglicht ist.

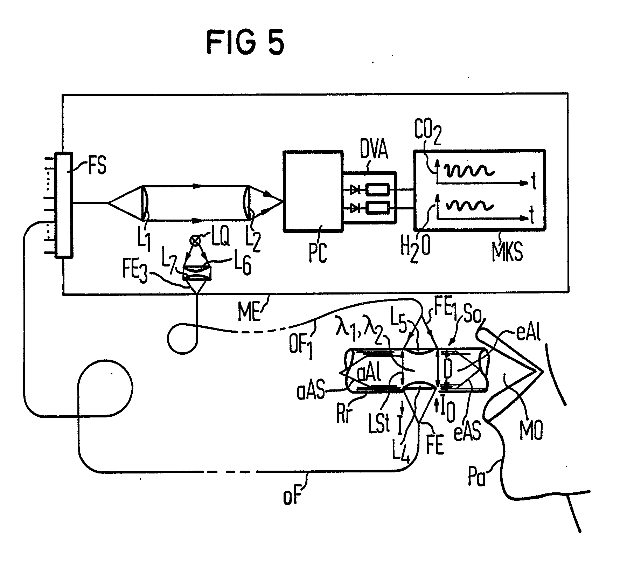

Die Ausführungsform nach Figur 5 unterscheidet sich von der Ausführungsform nach Figur 1 lediglich dadurch, daß für die Hinleitung des von der Lichtquelle LQ ausgesandten Lichts zum Atemluftstrom eAS bzw. aAS eine andere Faser $oF_1$ verwendet wird als für die Fortleitung des durch diesen Strom hindurchgegangenen Lichts. Bei dieser Ausführungsform nach Figur 5 fehlt der Strahlteiler Stl der Ausführungsform nach Figur 1. Das von der Lichtquelle LQ ausgesandte Licht wird von einer Linse $L_6$ kollimiert und von einer weiteren Linse $L_7$ auf ein Ende $FE_3$ der Faser $oF_1$ fokussiert und in diese Faser eingekoppelt. Anstelle des Spiegels Sp bei der Ausführungsform nach Figur 1 ist bei der Ausführrugnsform nach Figur 5 die Linse $L_5$ vorgesehen, die das aus dem anderen Ende $FE_1$ der Faser $oF_1$ ausgekoppelte Licht kollimiert.

## Ansprüche

1. Verfahren zur Überwachung des Atmens, wobei mittels einer von der ein- und ausgeatmeten Atemluft (eAl, aAl) durchströmten Sonde (So) eine sich mit der Konzentration wenigstens eines in der ausgeatmeten Atemluft (aAl) in anderer Konzentration als in der eingeatmeten Atemluft (eAl) enthaltenen Gases ändernde physikalische Größe laufend gemessen und auf Schwankungen hin überwacht wird, insbesondere für die Überwachung, besonders die intensive Überwachung eines Patienten, **dadurch gekennzeichnet,** daß die ein- und ausgeatmete Atemluft (eAl, aAl) einen Lichtstrahl (LSt) mit zumindest einer Wellenlänge ($\lambda_1$) durchströmt, bei welcher sich eine Größe (I) des in die Atemluft (eAl, aAl) eingestrahlten Lichts während des Durchgangs durch den Atemluftstrom (eAS, aAS) in Abhängigkeit von der Konzentration des Gases in der Atemluft (eAl, aAl) ändert, und daß bei zumindest der einen Wellenlänge ($\lambda_1$) die Größe (I) des durch den Atemluftstrom (eAS, aAS) hindurchgegangenen Lichts gemessen und auf Schwankungen hin überwacht wird.

2. Verfahren nach Anspruch 1, **dadurch gekennzeichnet,** daß als Größe des Lichts die Intensität (I) des durch den Atemluftstrom (eAS, aAS) hindurchgegangenen Lichts gemessen wird.

3. Verfahren nach Anspruch 2, **dadurch gekennzeichnet,** daß die Intensität (I) des durch den Atemluftstrom (eAS, aAS) hindurchgegangenen Lichts bei zumindest einer Wellenlänge ($\lambda_1$) gemessen wird, bei welcher das Gas das Licht in Abhängigkeit von seiner Konzentration in der Atemluft (eAl, aAl) mehr oder weniger absorbiert.

4. Verfahren nach Anspruch 3, **dadurch gekennzeichnet,** daß die Intensität (I) des durch den Atemluftstrom (eAS, aAS) hindurchgegangenen Lichts bei einer Wellenlänge ($\lambda_1$) gemessen wird, bei der das Gas das Licht absolut oder wenigstens relativ maximal absorbiert.

5. Verfahren nach Anspruch 4, **dadurch gekennzeichnet,** daß die Intensität (I) des durch den Atemluftstrom (eAS, aAS) hindurchgegangenen Lichts bei einer Wellenlänge ($\lambda_1$) im Infrarotbereich gemessen wird, bei der $CO_2$ das Licht absolut oder zumindest relativ maximal absorbiert.

6. Verfahren nach Anspruch 5, **dadurch gekennzeichnet,** daß die Intensität (I) bei der Wellenlänge 2,69 $\mu$m oder 2,70 $\mu$m gemessen wird.

7. Verfahren nach einem der vorhergehenden Ansprüche, **dadurch gekennzeichnet,** daß die Größe (I) des Lichts bei einer Wellenlänge ($\lambda_1$), bei der sich diese Größe (I) relativ stark mit der Konzentration des Gases in der Atemluft (eAl, aAl) ändert und bei einer weiteren Wellenlänge ($\lambda_2$), bei der sich die Größe des Lichts relativ schwach oder nioht mit der Konzentration des Gases in der Atemluft (eAl, aAl) ändert.

8. Verfahren nach Anspruch 6 und 7, **dadurch gekennzeichnet,** daß die Intensität des Lichts bei der weiteren Wellenlänge 2,60 $\mu$m gemessen wird.

9. Vorrichtung zur Durchführung eines Verfahrens nach einem der vorhergehenden Ansprüche, **gekennzeichnet durch** eine Lichtquelle (LQ) zur Erzeugung von Licht zumindest der einen Wellenlänge ($\lambda_1$) oder von Licht zumindest der einen und der anderen Wellenlänge ($\lambda_1,\lambda_2$), eine optische Faser (oF), die das von der Lichtquelle (LQ) ausgesandte und durch den Atemluftstrom (eAS, aAS) hindurchgegangene Licht einer entfernt von dem Atemluftstrom (eAS, aAS) angeordneten Meßeinrichtung (ME) zum Messen der Größe dieses Lichts bei zumindest der einen Wellenlänge ($\lambda_1$) oder zumindest der einen und weiteren Wellenlänge ($\lambda_1,\lambda_2$) zuleitet.

10. Vorrichtung nach Anspruch 9, **dadurch gekennzeichnet,** daß die das durch den Atemluftstrom (eAS, aAS) hindurchgegangene Licht dem Meßgerät (ME) zuleitende optische Faser (oF) zugleich die das Licht aus der Lichtquelle (LQ) dem Atemluftstrom zuleitende optische Faser ist, und daß der Atemluftstrom (eAS, aAS) zwischen einem nahe bei diesem Luftstrom angeordneten Ende (FE) dieser Faser und einem das aus diesem Ende (FE) ausgekoppelte Licht durch den Atemluftstrom (eAS, aAS) zu diesem Ende (FE) zurückreflektierenden Spiegel (Sp) strömt.

11. Vorrichtung nach Anspruch 10, **dadurch gekennzeichnet,** daß eine Fokussierungseinrichtung ($L_4$) vorgesehene ist, die das vom Spiegel (Sp) reflektierte Licht auf das nahe bei dem Atemluftstrom angeordnete Ende (FE) der optischen Faser (oF) fokussiert.

12. Vorrichtung nach Anspruch 11, **dadurch gekennzeichnet,** daß der Atemluftstrom (eAS, aAS) zwischen den nahe bei diesem Luftstrom angeordneten Ende (FE) der das durch den Atemluftstrom (eAS, aAS) hindurchgegangene Licht dem Meßgerät (ME) zuleitenden optischen Faser (oF) und einem nahe bei diesem Luftstrom (eAS, aAS) angeordneten Ende ($FE_1$) einer das Licht aus der Lichtquelle (LQ) diesem Strom zuleitenden anderen optischen Faser ($oF_1$) strömt, und daß eine Fokussierungseinrichtung ($L_5$, $L_4$) vorgesehen ist, die das aus dem Ende ($FE_1$) der anderen optischen Faser ($oF_1$) ausgekoppelte Licht auf das Ende (FE) der einen optishcen (oF) fokussiert.

13. Vorrichtung nach einem der Ansprüche 9 bis 12, **dadurch gekennzeichnet,** daß ein von der Atemluft durchströmtes Rohr (Rr) vorgesehen ist, das einen von dem Licht aus der Lichtquelle (LQ) durchstrahlten Atemluftstrom (eAS, aAS) bestimmten Durchmessers (D) definiert.

14. Vorrichtung nach einem der Ansprüche 9 bis 13, **dadurch gekennzeichnet,** daß die Entfernung des Meßgeräts (ME) vom Atemluftstrom (eAS, aAS) größer als 1m ist.

15. Vorrichtung nach einem der vorhergehenden Ansprüche, **dadurch gekennzeichnet,** daß die Entfernung der Lichtquelle (LQ) vom Atemluftstrom (eAS, aAS) größer als 1 m ist.

16. Vorrichtung nach Anspruch 14 oder 15 zur Durchführung eines Verfahrens nach einem der Ansprüche 5, 6 oder 8, **dadurch gekennzeichnet,** daß eine optische Faser ($oF_1$) aus einer Schwermetallfluorid-Glasfaser besteht.

17. Vorrichtung nach Anspruch 16, **dadurch gekennzeichnet,** daß die Glasfaser aus einer teflonummantelten Bariumfluorozirkonat-Glasfaser besteht.

18. Vorrichtung nach einem der Ansprüche 9 bis 17, **dadurch gekennzeichnet,** daß die Meßeinrichtung (ME) eine Einrichtung (PC) zur wellenlängenselektiven Registrierung mit mehreren Detektoren besteht, die wahlweise auf verschiedene Atemluftströme umschaltbar ist.

19. Verfahren zur Messung der Konzentration eines in Atemluft (eAl, aAl) enthaltenen Gases, insbsondere eines in der ausgeatmeten Atemluft (aAl) in anderer Konzentration als in der eingeatmeten Atemluft (eAl) enthaltenen Gases, **dadurch gekennzeichnet,** daß der Atemluftstrom der ein- und/oder ausgeatmeten Atemluft (eAl, aAl) von einem Lichtstrahl (LSt) mit zumindest einer Wellenlänge ($\lambda_1$) durchstrahlt wird, bei welcher sich eine Größe des Lichts beim Durchgang durch den Atemluftstrom (eAS, aAS) in Abhängigkeit von der Konzentration des Gases in der Atemluft (eAl, aAl) ändert, und daß bei zumindest der einen Wellenlänge ($\lambda_1$) die Größe des durch den Atemluftstrom (eAS, aAS) hindurchgegangenen Lichts gemessen wird.

# FIG 1

FIG 2

# FIG 3

Wellenlänge μm

# FIG 4

EP 0 309 666 A1

# FIG 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-3 243 776  (HONEYWELL AND PHILIPS MED, ELEC.) <br> * Zusammenfassung; Seite 1, Ansprüche 1,2; Seite 2, Zeile 29 - Seite 3, Zeile 10; Seite 3, Zeile 27 - Seite 5, Zeile 8; Abbildung * <br> --- | 1-6,9-13,19 | A 61 B  5/08 <br> G 01 N  21/35 |
| X | WO-A-8 606 638  (BRIGHAM AND WOMEN'S HOSPITAL) <br> * Zusammenfassung; Seite 3, Zeile 31 - Seite 4, Zeile 8; Seite 6, Zeile 30 - Seite 7, Zeile 12; Seite 9, Zeile 17 - Seite 11, Zeile 17; Seite 11, Zeile 28 - Seite 12, Zeile 26; Abbildungen 1-3 * <br> --- | 1-5,11,13,19 | |
| X | J. SCI. INSTRUM., Band 41, Nr. 12, Dezember 1964, Seiten 732-735, London, GB; D.W. HILL et al.: "A versatile infra-red gas analyser using transistors" <br> * Seiten 732-733, Abschnitte: 2. "General discription of the instrument" und 4. "optical filters"; Seite 735, Abschnitte 18: "performance of the analyses" und 19. "applications" * <br> --- | 1-5,7,13,19 | RECHERCHIERTE SACHGEBIETE (Int. Cl.4) <br><br> A 61 B <br> G 01 N |
| D,A | JOURNAL OF LIGHTWAVE TECHNOLOGY, Band LT-2, Nr. 5, Oktober 1984, Seiten 566-585; D.C. TRAN et al.: "Heavy metal fluoride glasses and fibers: A review" <br> * Seiten 566-585 * <br> ----- | 16,17 | |

Der vorliegende Recherchenbericht wurde für alle Patentansprüche erstellt

| Recherchenort | Abschlußdatum der Recherche | Prüfer |
|---|---|---|
| DEN HAAG | 30-12-1988 | RIEB K.D. |